# EUROPEAN PATENT SPECIFICATION

(11) **EP 0 493 985 B1**
(45) Date of publication and mention of the grant of the patent: **09.06.1999**
(21) Application number: 91312105.9
(22) Date of filing: 31.12.1991
(51) Int. Cl.: C07K 14/50, A61K 31/375, A61K 38/18, A61K 45/06

(54) **Wound healing compositions containing fibroblast growth factor and ascorbic acid**
Fibroblastwachstumsfaktor und Ascorbinsäure enthaltende Wundheilungszusammensetzungen
Compositions cicatrisantes pour les plaies contenant du facteur de croissance du fibroblaste et de l'acide ascorbique

(30) Priority: 02.01.1991 US 636843
(43) Date of publication of application: 08.07.1992
(73) Proprietor: JOHNSON & JOHNSON CONSUMER PRODUCTS, INC., Skillman, New Jersey 08558-9418 (US)
(72) Inventor: Geesin, Jeffrey C., Newton, PA 18940 (US); Gordon, Joel S., Princeton Junction, NJ 08550 (US)
(74) Representative: Fisher, Adrian John

(56) References cited:
- EP-A- 0 298 723
- EP-A- 0 324 387
- WO-A-87/01728
- MEDLINE ABSTRACT Number: 85160000 WEBBER R.J. et al: " Cell culture of rabbit meniscal fibrochondrocytes: proliferative and synthetic response to growth factors and ascorbate."
- ARCH.BIOCHEMISTRY AND BIOPHYSICS vol. 289, no. 1, 1991, pages 6 - 11; GESSIN J.C. ET AL: 'Modulation of collagen synthesis by growth factors: the role of ascorbate-stimulated lipid peroxidation.'

## Description

The present invention concerns compositions for treating a wound and increasing the rate of healing a wound containing fibroblast growth factor and ascorbic acid.

The process of wound healing can generally be divided into three overlapping stages. The first stage is the inflammation stage, which occurs over the first three days after the tissue has been wounded. The second stage is the granulation tissue formation stage, which occurs from the third day after wounding to the twelfth day. And the third stage is the matrix formation and remodeling stage, which occurs from about the third day to about the sixth month after wounding. During the inflammation stage the tissue injury results in the release of blood components into the wound site which activate a clotting cascade and provide a matrix for the influx of inflammatory cells. Afterwards, granulation tissue begins to form which consists of a dense population of fibroblasts, macrophages and neovasculature embedded in a loose matrix of collagen, fibronectin and hyaluronic acid, generally known as the Extra Cellular Matrix (ECM). Endothelial cells in the wound proliferate and form new blood vessels (angiogenesis) to supply the injured site with nutrients and oxygen. This nutrient supply is essential for synthesis, deposition and organization of the ECM.

Within hours of injury, re-epithelialization begins to restore the integrity of the damaged surface. The matrix formation and remodeling stage begins with the gradual dissolution of granulation tissue with devascularization and the loss of cells, fibronectin and type III collagen. The granulation tissue is replaced with connective tissue consisting of a framework of collagen and elastin fibers providing tissue strength and elastic properties, respectively. This framework then becomes saturated with proteoglycans and glycoproteins. Remodeling involves the synthesis of new collagen and the degradation of old collagen. The final outcome of matrix formation and remodeling is the scar tissue.

One of the most important aspects of wound healing is the rate at which a wound gains tensile strength. During wound healing, an increase in tensile strength parallels an increase in the collagen content of a wound. The gain in tensile strength correlates with the rate of collagen synthesis through the first ten weeks of wound healing.

Many studies have emphasized the importance of ascorbic acid in wound healing. Ascorbic acid is concentrated in healing wounds and its circulating levels are acutely diminished following skin injury. Also, ascorbic acid is essential for the growth and maintenance of connective tissue. It is a cofactor for several hydroxylating enzymes in the body, including prolyl hydroxylase and lysyl hydroxylase, enzymes that hydroxylate prolyl and lysyl residues, respectively, in the pro-collagen polypeptide to form hydroxyproline and hydroxylysine. Hydroxyproline is essential for maximum stability of the triple helix and, consequently, for secretion of pro-collagen from the cell. Hydroxylysine, on the other hand, participates in cross-link formation and serves as a site for covalent attachment of galactosyl or glucosyl-galactosyl residues during collagen biosynthesis. Ascorbic acid also stimulates collagen synthesis by stimulating lipid peroxidation.

Basic FGF (bFGF) is a potent mitogen for vascular endothelial cells, derived from either large vessels or capillaries. In addition, bFGF regulates the synthesis and deposition of various ECM components. Heparin sulfate, a structural component of the ECM, may function to stabilize FGF's since it protects both bFGF and acidic FGF (aFGF) from acid or heat inactivation and potentiates their mitogenic activity. aFGF and bFGF possess the same intrinsic biological activity but differ in their relative potencies.

FGF proteins have previously been described as being useful in increasing the rate of wound healing. See for instance, International Patent Application Publication No. WO 87/01728. It has been shown that FGF's role in such wound healing is to increase the formation of granulation tissue. However, such FGF stimulated wounds have not shown increased collagen content above that observed in untreated wounds or a specific increase relative to the increased cellularity of the granulation tissue. See for instance: McGee et al., J. Surgical Res. (1988), 45:145-153; Broadley, K.N. et al., Biotechn Ther (1989), 1:55; Davidson, J.M. et al., J. Cell Biochem (1988), Suppl. 12, Part A, p.147; and Davidson, J.M. et al., J.Cell Biol (1985), 100:1219-1222.

Webber et al. in Journal of Orthopaedic Research vol. 3(1) pp. 36-42 (9185) describe the proliferative and synthetic response of cell cultives of rabbit meniscal fibrochondrocytes to growth factors such as FGF and ascorbate. It was found that both FGF and ascorbate decreased 35-sulfate incorporation, whereas only ascorbate was found to alter the amount of sulfated glycosaminoglycan in the pericellular coat. The effect of FGF and ascorbate in combination was not studied.

The present invention provides a wound healing composition comprising from 1 x 10⁻⁷% to 0.1% w/w of a fibroblast growth factor (FGF) and from 0.001% to 5.0% w/w of ascorbic acid.

The FGF may be human basic FGF or human acidic FGF. The ascorbic acid may be L-ascorbic acid or a derivative, analog or isomer thereof. A method for healing a wound in a patient comprises applying to the wound a wound healing amount of the composition of the present invention.

Ascorbic acid, also known as L-ascorbic acid or vitamin C, is an unsaturated aliphatic sugar acid and is the gamma-lactone of a hexonic acid having an enediol structure at carbon atoms 2 and 3. It is a very unstable compound and readily undergoes oxidation to the saturated dehydroascorbic acid. Ascorbate refers to a salt or ester of ascorbic acid. There are known derivatives, analogs or isomers of ascorbic acid. Examples of such derivatives, analogs or isomers are D-ascorbic acid; D-isoascorbic acid; L-dehydroascorbic acid; ascorbic acid ethers; ascorbic acid esters, such as lipid esters; and salts thereof of, such as sodium, potassium, phosphate and magnesium salts. As used herein, the phrase "ascorbic acid" refers to L-ascorbic acid, any derivatives, analogs or isomers of L-ascorbic acid that also are capable of stimulating lipid peroxidation and any salts or esters thereof.

FGF proteins are members of a family of single-chained proteins of 14-18 kDa, which bind tightly to heparin. Two well defined forms of FGF are acidic FGF and basic FGF. aFGF is a protein of 140 amino acids (15.6 kDa) with a pI of 5.6-6.0. bFGF is a protein of 146 amino acids (16.4 kDa) with a pI of 9.6. In most systems examined, bFGF is more stable and exhibits a ten-fold greater potency than aFGF. Both forms of FGF bind to the same receptor. FGF's are mitogenic for cells of mesodermal origin, such as fibroblasts, vascular endothelial cells, vascular smooth muscle cells, myoblasts, keratinocytes, chondrocytes and osteoblasts.

FGF's may be isolated from natural sources, such as brain and retina for aFGF or brain and pituitary for bFGF. Acidic and basic FGF may also be obtained by recombinant DNA methods of production, such as those described in International Patent Application Publication No. WO 87/01728.

As used herein "FGF" refers to acidic FGF or basic FGF, preferably of human origin, as well as any biologically active analogs, fragments or chemical synthesized derivatives thereof. The term "analog" of FGF refers to any polypeptide having a substantially identical amino acid sequence to that of FGF in which one or more amino acids has been substituted with chemically similar amino acids. The term "analog" shall also include any polypeptide which has one or more amino acid deleted from or added to the polypeptide, but which still retains a substantial amino acid sequence homology to the polypeptide. A substantial sequence homology is any homology greater than 50%. Analogs of FGF are described in European Patent Application Publication Nos. EP 0 320 148, EP 0 298 723, and EP 0 281 822. The phrase "fragment of FGF" refers to any shorter version of the FGF polypeptide, having at least 10 amino acids residues and having the same bioactivity as naturally occurring FGF. The phrase "chemical derivative" refers to any polypeptide derived from FGF in which one or more amino acids have been chemically derivatized synthetically by reaction of functional side groups of the amino acids (i.e., it is derived from the parent FGF molecule by one or more steps).

The present invention provides a wound healing composition comprising a mixture of FGF, preferably purified, and ascorbic acid. The term "purified" as used herein refers to FGF which, prior to mixing with the ascorbic acid, is 95% or greater, by weight, FGF, i.e., it is substantially free of other proteins, lipids and carbohydrates with which it is naturally associated. A purified FGF protein preparation will generally yield a single major band on polyacrylamide gel electrophoresis.

The FGF and ascorbic acid are present in the compositions of the present invention in amounts that are sufficient to enhance the rate of wound healing in a patient by increasing the tensile strength of a wound or increasing the rate of collagen synthesis at a wound site or the amount of collagen deposited at a wound site. Such amounts are referred to herein as "an effective amount". As is well known, particularly in the medicinal arts, effective amounts of medicinal agents vary with the particular agent employed, the condition being treated and the rate at which the composition containing the medicinal agent is eliminated from the body, as well as varying with the animal in which it is used, and the body weight of the animal. Thus, an effective amount is that amount which in a composition of this invention provides a sufficient amount of FGF and ascorbic acid to provide an increase in the rate of wound healing in the body of the treated animal.

The compositions of the present invention may be used in the form of an aqueous medicinal composition such as a buffered solution, gel, cream, suspension or dispersion. The compositions may also be in the form of an ointment. An effective wound healing amount of the FGF in an aqueous composition is in the range of from about 1 ng to about 1 mg per milliliter of the aqueous formulation. Preferably, the FGF concentration is in the range 100 ng-10 micrograms per milliliter. The concentration of the ascorbic acid in an aqueous formulation is in the range 10 micrograms/ml to 50 mg/ml, preferably 0.5 mg/ml to 5.0 mg/ml. Expressed on a weight percentage basis (weight of ingredient per total weight of formulation), the ascorbic acid is present in the formulation at 0.001% to 5.0% (w/w), preferably 0.05% to 0.5%. The exact percentage of the ascorbic acid will depend on the particular ascorbic acid used and its potency. Obviously, the more potent the ascorbic acid the less that will be needed in the formulation. The growth factor is present at 1x10⁻⁷% to 0.1% (w/w), preferably 1x10⁻⁵% to 1x10⁻³%.

The compositions of the present invention contain pharmaceutically acceptable FGF and ascorbic acid in an intimate mixture. As used herein, the phrase "pharmaceutically acceptable" means that the material so described may be used for treatments in or on humans or other mammals without causing ill effects, such as toxicity, blistering or whitening of mucosa or skin tissues. The phrase "intimate mixture" is used herein to mean that the components of the composition are mixed substantially uniformly so that none of those components are localized. A minor amount of agitation immediately prior to use may be required for some liquid compositions of this invention to achieve an intimately mixed state when used.

The compositions of the present invention may be formulated in any number of ways depending on whether an aqueous solution, cream or ointment is desired and whether it will be used internally or topically on the surface of the skin or in the eye or oral mucosa. A suitable water miscible (substantially oil free and fat free) liquid carrier is a mixture of an alcohol, such as ethyl alcohol or isopropyl alcohol, at 25-75% by weight, and the balance of a liquid glycol, such as ethylene glycol or polyethylene glycol.

Compositions formulated as a cream may contain a cream stabilizer, such as xanthan gum; an emulsifier, preferably a non-ionic emulsifier; at least one liquid and one solid hydrophobic material selected from the liquid and solid fatty acids, fatty alcohols, fatty acid esters, pharmaceutical grades of waxes and hydrocarbons, the latter ranging from liquids, through semisolids such as petrolatum, to solids and the like; a preservative; and water.

The compositions of the present invention may be useful in eye drop formulations, eye gels, eye creams, liposomes or micell formulations, aqueous vehicles for soaking gauze dressings, burn dressings, artificial skins, sutures and staple coatings, salves or creams, gel formulations, foams and the like. Additional materials such as buffers, preservatives, tonicity adjusting agents, polymers for adjusting viscosity or for use as extenders, and excipients may be used in the compositions. Specific illustrative examples of such other materials include phosphate, citrate, or borate buffers; thimerosal, sorbic acid, methyl or propyl paraben and chlorobutanol preservatives; sodium chloride and/or sugars to adjust the tonicity; and excipients such as mannitol, lactose or sucrose.

Methods for increasing the rate of healing a wound comprise applying or administering a composition of the present invention directly to an external or internal wound. The composition is permitted to remain in contact with the wound for a period of time sufficient to increase the tensile strength or the rate of cell growth, collagen deposition or collagen synthesis at the wound site. Such methods include incorporating any composition of the present invention into a cream formulation or soaking a gauze dressing with an aqueous solution of the composition and then applying the cream or soaked gauze to a wound site. Additionally, sutures or staples may be coated or soaked with the composition and used to close an open wound.

The types of wounds that may be healed using the compositions of the present invention are those which result from any accidental or medical injury which causes epithelial damage such as ophthalmic wounds, such as those which result from corneal ulcers, radiokeratotomy, corneal transplants, epikeratophakia and other surgically induced wounds in the eye; and cutaneous wounds such as incisions, deep surgical wounds, burn wounds, donor site wounds from skin transplants and ulcers (cutaneous, decubitis, venous stasis and diabetic). Additionally, dermatological conditions in which the skin has been damaged, such as psoriasis, sunburn and skin rashes, may be treated with the compositions of the present invention. The compositions may be applied to the wound site either topically or internally depending on the type of wound.

Ascorbic acid has been shown to stimulate collagen synthesis through induction of lipid peroxidation leading to increased transcription of the collagen genes. The mechanism by which lipid peroxidation stimulates collagen transcription is unknown, however, an alteration of cell membranes may affect the activity of serum growth factors that leads to a change in gene expression.

To test this hypothesis, we treated dermal fibroblasts with FGF in the presence of lipid peroxidation stimulating (200 micromolar) and nonstimulating (1 micromolar) concentrations of ascorbic acid. FGF affected collagen synthesis in the presence of 200 micromolar ascorbic acid producing stimulation (0.4-2.0 ng/ml). These results indicate that ascorbate-induced lipid peroxidation can modulate the activity of FGF.

The examples which follow are presented to illustrate the subject invention. The invention is not to be considered limited by these examples, but only by the appended claims.

### EXAMPLE 1

Ascorbic acid has been shown to stimulate collagen synthesis in cultured human dermal fibroblasts through stimulation of the expression of collagen genes for the α1(I), α2(I), α1(III), and α1(IV) chains without changing the rate of intracellular degradation. This process has also been shown to involve the induction of lipid peroxidation by ascorbic acid. The process by which lipid peroxidation leads to a stimulation of collagen synthesis is unknown.

We chose to test the activities of FGF on human dermal fibroblasts in the presence of lipid peroxidation stimulating (200 µM) and nonstimulating (1 µM) concentrations of ascorbic acid. FGF has been shown to effect cell proliferation, cell differentiation or matrix synthesis, which are mediated through cell surface receptors. Therefore, we tested the ability of ascorbic acid to alter the response of dermal fibroblasts to FGF.

### METHODS AND PROCEDURES

Materials - L-Ascorbic acid was purchased from Sigma Chemical Company and all media containing it was made fresh daily due to its instability in solution. FGF was purchased from Collaborative Research and was from bovine pituitary.

Cell Culture-Primary cultures of newborn human dermal fibroblasts (American Type Culture Collection) were grown and treated as previously described for the determination of collagen synthesis (see, Geesin, J.C. et al., J. Invest. Dermatol. (1988) 90:420-424). Briefly, cells were seeded at 100,000 per 35 mm diameter tissue culture dish and grown to confluence in Dulbecco's modified Eagle's medium (DMEM) buffered with 24 mM sodium bicarbonate and 25 mM HEPES and supplemented with 20% heat-inactivated dialyzed calf serum (HIDCS) (Grand Island Biological). Plates were then washed three times with phosphate-buffered saline (PBS) and treated for three days in DMEM containing 0.5% HIDCS with the appropriate concentration of ascorbic acid and FGF. Cells were incubated for the final six hours with the test media containing 20 µCi/ml 2,3-[3H]-1-proline (Amersham).

Measurement of Collagen Synthesis - By a previously described protocol (see Geesin et al., supra), media from treated plates was combined with a solution of protease inhibitors and stored at -20°C until analysis. The cells were counted on a Coulter counter after trypsinization. The medias were then dialyzed extensively versus 0.33 M calcium acetate, 25 mM Tris, pH 7.4, 0.02% sodium azide with 0.6 mM N-ethylmaleimide. Measured aliquots of the samples were combined with bovine serum albumin and digested with highly purified collagenase (Collagenase Form III, Advanced Biofactures, Inc.) by an incubation at 37°C for 4 hours. The samples were then precipitated with 25% trichloroacetic acid, 1.25% tannic acid and centrifuged at 12,000g for 3.5 minutes. The collagenase solubilized counts were determined by diluting the supernatent with Liquiscint (National Diagnostics) and quantifying on an Intertechnique SL 4000 scintillation spectrometer. The radioactivity of an undigested sample was similarly quantified. The collagenase soluble counts were used to determine the amount of newly synthesized collagen protein and are reported as collagenase soluble counts per cell.

### RESULTS

FGF in the presence of ascorbate concentrations capable of supporting proline hydroxylation, but incapable of stimulating lipid peroxidation, has a minimal effect on collagen synthesis in fibroblasts. In the cultures treated with 1 µM ascorbic acid, no statistically significant change was seen in either collagen synthesis/cell or percent collagen synthesis. However, in the presence of 200 µM ascorbic acid, a concentration capable of stimulating lipid peroxidation, collagen synthesis was increased and then decreased depending upon the concentration of FGF used. At 0.4 ng/ml FGF, collagen synthesis/cell was 50% greater than that seen in the absence of FGF. At concentrations of FGF above 10 ng/ml, collagen synthesis/cell was significantly less than that seen in the absence of FGF.

Table 1 lists the results (as collagen cpm/1000 cells).

**TABLE 1**

| EFFECTS OF ASCORBATE AND FIBROBLAST GROWTH FACTOR ON COLLAGEN SYNTHESIS | | | | | |
|---|---|---|---|---|---|
| Treatment | | Plate Number | | | |
| | | 1 | 2 | 3 | avg (std) |
| Control | A | 3.295 | 3.723 | 2.845 | |
| | B | 2.345 | 2.191 | 2.845 | 2.874 (0.302) |
| Control + FGF | A | 3.950 | 3.804 | 3.968 | |
| | B | 4.324 | 3.142 | 3.564 | 3.792 (0.213) |
| Control + Ascorbate | A | 47.295 | 54.710 | 46.765 | |
| | B | 41.486 | 48.513 | 46.598 | 47.561 (2.243) |
| Control + FGF + Ascorbate | A | 66.485 | 69.257 | 66.248 | |
| | B | 67.357 | 81.342 | 67.501 | 69.698 (3.058) |

### DISCUSSION

In this experiment, we tested the ability of ascorbate-induced lipid peroxidation to interfere with receptor mediated effects of FGF. The effect of FGF in low (1 µM) and high (200 µM) concentrations of ascorbic acid was compared. These concentrations were selected so the effects of lipid peroxidation could be differentiated. In previous studies, 200 µM ascorbate was shown to stimulate both lipid peroxidation and collagen synthesis, whereas at 1 µM ascorbic acid, there was shown to be little or no induction of lipid peroxidation. We chose to use 1 µM ascorbate because, at this concentration, the activity of the enzyme prolyl hydroxylase is not limited by the absence of ascorbate. Therefore, under these conditions, all the collagen molecules are properly hydroxylated which permits the formation of the characteristic triple helical conformation of collagen which is necessary for secretion. In this way, under the conditions tested in these experiments, the effect of ascorbic acid induced lipid peroxidation can be determined versus controls which do not have a deficiency in the rate of hydroxylation of important proline residues.

FGF stimulates collagen synthesis only at ascorbate concentrations that produce lipid peroxidation. Therefore, FGF stimulation of collagen synthesis appears to occur only in the presence of ascorbate-induced lipid peroxidation and therefore represents a cell membrane associated mechanism capable of being manipulated by ascorbate-induced lipid peroxidation. FGF has been implicated in wound healing through its ability to cause rapid neovascularization and fibroplasia in skin (see, Folkman, J. et al., Science (1987) 235:442-447) which are assumed to be receptor mediated, although there was previously no evidence that collagen synthesis was stimulated. The recently described interactions between FGF and the extracellular matrix have implied that this growth factor plays an integral role in the wound healing response. Our findings indicating that FGF can stimulate collagen synthesis in a lipid peroxidation dependent manner demonstrates that the addition of an activator of lipid peroxidation such as ascorbate, will enhance FGF stimulation of tissue repair.

### Example 2

A general cream formulation containing recombinant human bFGF and L-ascorbic acid is set forth below in Table 2. The listed ingredients may be formulated according to standard procedures known in the pharmaceutical formulation art.

**Table 2**

| Ingredient | %(w/w) |
|---|---|
| bFGF | 1x10⁻⁵ - 1x10⁻³ |
| L-Ascorbic Acid | 0.05 - 0.5 |
| Xanthan gum | 0.1 - 1.0 |
| Non-ionic emulsifier | 1.0 - 10.0 |
| Combination of liquid and solid fatty acids, fatty alcohols, fatty acid esters and/or other pharmaceutically acceptable hydrophobic materials | 15.0 - 50.0 |
| Preservative(s) | 0.05 - 1.0 |
| Water | balance to 100.0 |

Optionally, minor amounts of other commonly used cosmetic adjuvants, additives and the like for use as humectants, sequestering agents, dyes, perfume oils and sunscreens may also be included.

The present invention has been described herein with reference to certain preferred embodiments and examples . Obvious variations within the scope of the accompanying claims may appear to those skilled in the art.

## Claims

1. A wound healing composition comprising from 1 x 10⁻⁷% to 0.1% w/w of a fibroblast growth factor (FGF) and from 0.001% to 5.0% w/w of ascorbic acid.

2. A composition according to claim 1 wherein the FGF is human basic FGF.

3. A composition according to claim I wherein the FGF is human acidic FGF.

4. A composition according to claim 1, 2 or 3, wherein the ascorbic acid is L-ascorbic acid.

5. A composition according to any preceding claim, comprising from 1 x 10⁻⁵% to 1 x 10⁻³% w/w of said FGF.

6. A composition according to any preceding claim, comprising from 0.05% to 0.5% w/w of ascorbic acid.

## Patentansprüche

1. Eine Wundheilungszusammensetzung umfassend von 1 x 10⁻⁷ % bis 0,1 % Gew./Gew. eines Fibroblastenwachstumsfaktors (FGF) und von 0,001 % bis 5,0 % Gew./Gew. Ascorbinsäure.

2. Eine Zusammensetzung nach Anspruch 1, wobei der FGF basischer FGF vom Menschen ist.

3. Eine Zusammensetzung nach Anspruch 1, wobei der FGF saurer FGF vom Menschen ist.

4. Eine Zusammensetzung nach Anspruch 1, 2 oder 3, wobei die Ascorbinsäure L-Ascorbinsäure ist.

5. Eine Zusammensetzung nach einem der vorhergehenden Ansprüche umfassend von 1 x 10⁻⁵ % bis 1 x 10⁻³ % Gew./Gew. des FGF.

6. Eine Zusammensetzung nach einem der vorhergehenden Ansprüche umfassend von 0,05 % bis 0,5 % Gew./Gew. Ascorbinsäure.

## Revendications

1. Composition cicatrisante pour blessure comprenant de 1 x 10⁻⁷% à 0,1% p/p d'un facteur de croissance de fibroblaste (FGF) et de 0,001% à 5,0% p/p d'acide ascorbique.

2. Composition selon la revendication 1, dans laquelle le FGF est un FGF humain basique.

3. Composition selon la revendication 1, dans laquelle le FGF est un FGF humain acide.

4. Composition selon la revendication 1, 2 ou 3, dans laquelle l'acide ascorbique est l'acide L-ascorbique.

5. Composition selon l'une quelconque des revendications précédentes, comprenant de 1 x 10⁻⁵% à 1 x 10⁻³% p/p dudit FGF.

6. Composition selon l'une quelconque des revendications précédentes, comprenant de 0,05% à 0,5% p/p d'acide ascorbique.
